(19) **Europäisches Patentamt**
**European Patent Office**
**Office européen des brevets**

(11) **EP 4 700 359 A1**

(12) **EUROPEAN PATENT APPLICATION**
published in accordance with Art. 153(4) EPC

(43) Date of publication:
**25.02.2026 Bulletin 2026/09**

(21) Application number: **24792524.1**

(22) Date of filing: **05.04.2024**

(51) International Patent Classification (IPC):
**G01N 3/00** (2006.01)     **G01N 3/08** (2006.01)
**G01N 19/00** (2006.01)

(52) Cooperative Patent Classification (CPC):
**G01N 3/08; G01N 3/00; G01N 3/02; G01N 3/42;
G01N 19/00; H05K 3/22;** H05K 2203/162

(86) International application number:
**PCT/JP2024/014015**

(87) International publication number:
**WO 2024/219249 (24.10.2024 Gazette 2024/43)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **19.04.2023 JP 2023068328**

(71) Applicant: **RESONAC CORPORATION**
**Tokyo 105-7325 (JP)**

(72) Inventors:
• **TAKAHASHI Masaki**
**Tokyo 105-7325 (JP)**
• **OTAKE Shunsuke**
**Tokyo 105-7325 (JP)**
• **NOMA Hirokazu**
**Tokyo 105-7325 (JP)**

(74) Representative: **Hoffmann Eitle**
**Patent- und Rechtsanwälte PartmbB
Arabellastraße 30
81925 München (DE)**

(54) **PRINTED CIRCUIT BOARD SUBSTRATE RELIABILITY TESTING METHOD**

(57)     A reliability test method for a printed wiring board substrate includes measuring a load at which a crack occurs in the printed wiring board substrate using a nanoindenter.

[Fig. 6]

## Description

Technical Field

The present embodiment relates to a reliability test method for a printed wiring board substrate.

Background Art

In recent years, in electronic devices such as computers, the capacity and speed of signals used have been increased, and semiconductor packages used in these electronic devices have also been highly integrated and functionalized.

In addition, semiconductor packages are required to be adapted to various temperature conditions depending on devices to be used, installation environments, and the like, and ensuring long-term reliability under temperature fluctuations is becoming increasingly important.

As a printed wiring board substrate, a substrate containing a resin component as an insulating material is used. However, due to a difference in thermal expansion coefficient between the resin component and an inorganic component such as a conductor layer or a semiconductor chip, stress may be generated when the temperature changes, and a crack may occur in the printed wiring board substrate. In particular, since the number of build-up layers tends to increase, the stress caused by the difference in thermal expansion coefficient is becoming large, and the problem of cracks occurring due to temperature changes has become prominent, in recent years.

As a reliability test method for a printed wiring board substrate, a temperature cycle test is generally performed (for example, see Patent Literature 1).

Citation List

Patent Literature

Patent Literature 1: JP 2006-093618 A

Summary of Invention

Technical Problem

The temperature cycle test is an effective test method for confirming long-term reliability of a test object with respect to a temperature change by repeating heating and cooling. On the other hand, since the temperature cycle test requires a period of several weeks to several months to obtain a test result, a simpler reliability evaluation method is desired.

In view of the above-described circumstances, a problem of the present embodiment is to provide a reliability test method for a printed wiring board substrate that can be easily performed.

Solution to Problem

As a result of diligent studies to solve the above problem, the present inventors have found that the above problem can be solved by the following embodiment.

That is, the present embodiment relates to the following items [1] to [6].

[1] A reliability test method for a printed wiring board substrate, the method including
measuring a load at which a crack occurs in the printed wiring board substrate, using a nanoindenter.
[2] The reliability test method for a printed wiring board substrate according to item [1] above, in which the printed wiring board substrate includes a resin cured product and a glass cloth.
[3] The reliability test method for a printed wiring board substrate according to item [1] or [2] above, in which the printed wiring board substrate has two or more layers each including a resin cured product and a glass cloth.
[4] The reliability test method for a printed wiring board substrate according to item [2] above, in which an indenter of the nanoindenter is pushed into the resin cured product.
[5] The reliability test method for a printed wiring board substrate according to any of items [1] to [4] above, in which the indenter of the nanoindenter is pushed into a cross-sectional surface of the printed wiring board substrate.
[6] The reliability test method for a printed wiring board substrate according to any of items [1] to [5] above, in which the indenter of the nanoindenter is a Berkovich indenter.

Advantageous Effects of Invention

According to the present embodiment, it is possible to provide a reliability test method for a printed wiring board substrate that can be easily performed.

Brief Description of Drawings

Fig. 1 is a graph for explaining a load-displacement curve.
Fig. 2 is a graph for explaining a load-contact stiffness curve.
Fig. 3 is a schematic view for explaining positions observed by an optical microscope in a liquid bath temperature cycle test.
Fig. 4 is a schematic view for explaining a position where a test piece is sampled and a test region for a fracture test by a nanoindenter.
Fig. 5 is a diagram for explaining a method of analyzing a load-contact stiffness curve.
Fig. 6 is a graph representing a correlation between crack occurrence loads W1 and crack occurrence rates after temperature cycle tests (1,000 cycles), made based on results of Examples.

Description of Embodiments

The action mechanism described in the present specification is inferential and does not limit the mechanism of providing an effect of the present embodiment.
An aspect in which matters described in the present specification are arbitrarily combined is also included in the present embodiment.

Reliability test method for printed wiring board substrate

A reliability test method for a printed wiring board substrate according to the present embodiment measures a load at which a crack occurs in the printed wiring board substrate using a nanoindenter.
According to the reliability test method of the present embodiment, since a long period of time is not required for the test unlike a temperature cycle test, the reliability of the printed wiring board substrate can be evaluated in a simple manner.

Nanoindenter

The nanoindenter used in the reliability test method of the present embodiment is a microhardness tester that pushes a minute indenter into a surface of a sample to a depth ranging from a few nanometers to several tens of micrometers, and can measure the relationship between the displacement amount and the load at that time.

Printed wiring board substrate

The printed wiring board substrate, which is a test object in the reliability test method of the present embodiment, is not particularly limited, and a known printed wiring board substrate can be used as a test object.
An insulating layer included in the printed wiring board substrate may be a layer that contains a resin and contains no glass cloth, or may be a layer that contains a resin and a glass cloth. A layer including a resin cured product and a glass cloth is preferable as the layer that contains a resin and a glass cloth. In the present specification, the "resin cured product" means a cured product that includes at least a resin and includes no glass cloth, and is preferably a cured product of a thermosetting resin composition containing a thermosetting resin.
The thermosetting resin composition may contain, as a component other than the thermosetting resin, a curing agent, a curing accelerator, an inorganic filler, and the like, if needed.
The insulating layer included in the printed wiring board substrate may be a single layer or multiple layers.
A specific example of the test object for the reliability test method of the present embodiment is, for example, a printed wiring board substrate having two or more layers each including a resin cured product and a glass cloth.
The printed wiring board substrate may have a member other than the insulating layer which may be generally included in the printed wiring board substrate. Examples of the member other than the insulating layer include a conductor layer formed on a surface of the substrate, a conductor layer formed between insulating layers, and a semiconductor chip mounted on a surface of the substrate.

The content of the resin cured product in the layer containing the resin cured product and the glass cloth is not particularly limited, and may be 25% to 75% by mass, may be 30% to 70% by mass, and may be 35% to 65% by mass, for example.

The thickness per layer of the layer containing the resin cured product and the glass cloth is not particularly limited, and may be 200 to 1,900 $\mu$m, may be 500 to 1,800 $\mu$m, and may be 800 to 1,700 $\mu$m, for example.

When the substrate has two or more layers each containing the resin cured product and the glass cloth, the number of layers each containing the resin cured product and the glass cloth is not particularly limited, and may be 2 to 19 layers, may be 5 to 18 layers, and may be 8 to 17 layers, for example.

Test conditions

Next, test conditions of a fracture test using the nanoindenter will be described.

In the following description, a test for measuring a load at which a crack occurs in a printed wiring board substrate using a nanoindenter is referred to as the "fracture test".

In the following description, a load at which a crack occurs in a printed wiring board substrate measured in the fracture test is referred to as a "crack occurrence load".

Although the position of the printed wiring board substrate subjected to the fracture test by the nanoindenter is not particularly limited, it is preferable to select a site where cracks are likely to occur in a temperature cycle test, for example. The site where cracks are likely to occur in a temperature cycle test is a site where stress is likely to be generated in the printed wiring board substrate. Therefore, the crack occurrence load is easily obtained in the range of the fracture test by the nanoindenter.

In a plan view of the printed wiring board substrate, an example of the site where the fracture test is performed is preferably an end portion of the printed wiring board substrate from the viewpoint of easily obtaining the crack occurrence load. Further, from the viewpoint of easily obtaining the crack occurrence load, the indenter of the nanoindenter is preferably pushed into a cross sectional surface of the printed wiring board substrate. Since the printed wiring board substrate is obtained by cutting out the outer shape thereof in a plan view to have a desired shape, an end surface of the printed wiring board substrate corresponds to the cross sectional surface.

In a cross sectional view of the printed wiring board substrate, an example of a site where the fracture test is performed is substantially a central portion of the printed wiring board substrate in the thickness direction.

That is, the site where the fracture test is performed is preferably a cross sectional surface of an end portion of the printed wiring board substrate in a plan view, and is preferably substantially a central portion of the printed wiring board substrate in a cross sectional view.

In performing the fracture test, it is preferable to process the printed wiring board substrate by cutting or the like to form a test piece including a region to be subjected to the fracture test, in order to mount same on the nanoindenter.

The surface of the test piece into which the indenter of the nanoindenter is pushed is preferably smoothed in advance by polishing before or after being sampled as a test piece, from the viewpoint of suppressing occurrence of variation in test results due to unevenness of the surface. Although the polishing method is not particularly limited, mechanical polishing or mechanochemical polishing is preferable.

If necessary, the test piece sampled from the printed wiring board substrate may be subjected to a pretreatment before the fracture test. Examples of the pretreatment include heat treatment and cooling treatment. By performing heat treatment and cooling treatment as the pretreatment, internal stress is accumulated in the test piece, and the crack occurrence load is easily obtained in the range of the fracture test by the nanoindenter.

Conditions of fracture test

Although the type of the indenter of the nanoindenter is not particularly limited, a Berkovich indenter is preferable from the viewpoint of versatility. The Berkovich indenter is an indenter in which the shape of a surface pushed into the test piece is a triangular pyramid (with a ridge interval of 115°). The type and the ridge interval of the indenter are not particularly limited, and a Berkovich indenter (a ridge interval of 100°), a Rockwell indenter, a Vickers indenter, a Knoop indenter, or a Shore indenter may be used.

Since the indenter of the nanoindenter is minute, when a substrate including a plurality of kinds of materials such as a layer containing a resin cured product and a glass cloth is to be tested, the obtained load-displacement curve may change depending on the position where the indenter is pushed into.

In a case where the object to be measured is a substrate having a layer containing a resin cured product and a glass cloth, when the indenter is pushed into the glass cloth or into the resin cured product at a position close to the glass cloth, a crack of the glass cloth is likely to occur. Meanwhile, when the indenter is pushed into the resin cured product at a position appropriately distant from the glass cloth, a crack at an interface between the glass cloth and the resin cured product and a crack of the resin cured product are likely to occur, and when the indenter is pushed into the resin cured product at a position further distant from the glass cloth, only a crack of the resin cured product is likely to occur.

In a temperature cycle test of the substrate having the layer containing the resin cured product and the glass cloth, since cracks at the interface between the resin cured product and the glass cloth and cracks of the resin cured product are likely to become a problem, it is preferable to adjust the position where the indenter is pushed into to a position where a crack at the interface between the resin cured product and the glass cloth and a crack of the resin cured product are likely to occur.

In view of the above, the indenter is preferably pushed into the resin cured product, and is preferably pushed into the resin cured product at a position appropriately distant from the glass cloth. On the surface into which the indenter is pushed, the shortest distance between the glass cloth and the center position on the resin cured product where the indenter is pushed into may be 1 to 10 $\mu$m, may be 2 to 8 $\mu$m, and may be 3 to 7 $\mu$m, for example.

The position where the indenter is pressed against can be adjusted by an optical microscope and a micrometer attached to the nanoindenter.

The indentation speed of the indenter is not particularly limited, but may be 15 to 55 mN/s, may be 25 to 50 mN/s, and may be 35 to 45 mN/s, for example.

The temperature at which the fracture test is performed is not particularly limited, but may be 5°C to 50°C, may be 10°C to 40°C, and may be 20°C to 30°C from the viewpoint of workability.

Although the atmosphere in which the fracture test is performed is not particularly limited, the fracture test is preferably performed in air from the viewpoint of workability.

Analysis conditions

A load-displacement curve is obtained by the fracture test described above.

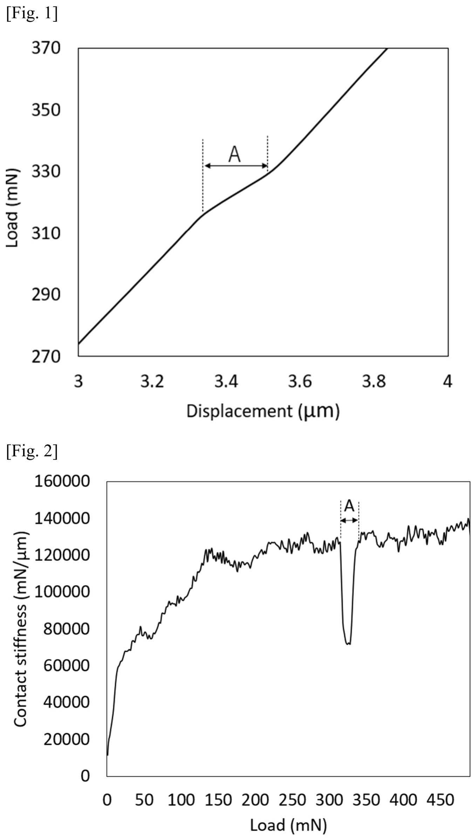

One example of the load-displacement curve is shown in Fig. 1.

As in the load-displacement curve shown in Fig. 1, the load as a whole tends to increase as the indentation depth of the indenter into the test piece, that is, the displacement increases; however, a region in which the increase of the load becomes gentle is generated as in the region A shown in Fig. 1 due to a crack of the test piece.

Fig. 2 shows a curve (hereinafter, also referred to as the "load-contact stiffness curve") obtained by plotting the contact stiffness, which is calculated as the slope of the load-displacement curve shown in Fig. 1, on the vertical axis and plotting the load on the horizontal axis. As shown in Fig. 2, the region A in Fig. 1 appears as a downward peak at which the contact stiffness decreases. Hereinafter, this peak is referred to as the "crack occurrence peak".

As described later in Examples, the load at which the crack occurrence peak appears correlates with the crack occurrence rate in a temperature cycle test. Therefore, the reliability of the printed wiring board substrate can be evaluated by measuring the load (hereinafter also referred to as the "crack occurrence load") at which the crack occurrence peak appears. For example, by grasping the correlation between the crack occurrence load and the crack occurrence rate in the temperature cycle test in advance, the crack occurrence rate in the temperature cycle test can be predicted from the crack occurrence load.

When a plurality of crack occurrence peaks appears, a threshold value may be set for the magnitude of the crack occurrence peak, and a crack occurrence peak with magnitude equal to or greater than a predetermined value may be used as an index for reliability evaluation. The threshold value of the magnitude of the crack occurrence peak may be, for example, a contact stiffness decrease rate (%) obtained from the following equation (1).

$$(1): \text{Contact stiffness decrease rate } (\%) = (S1 - S2) \times 100/S1$$

In the equation, S1 means the contact stiffness obtained when the contact stiffness starts to decrease in the crack occurrence peak, and S2 means the contact stiffness at the peak top of the crack occurrence peak.

Incidentally, the "contact stiffness obtained when the contact stiffness starts to decrease" can be determined by the method described in Examples, for example. In addition, the "peak top" means the top of the crack occurrence peak (the point representing the minimum value of the contact stiffness in the peak).

After the crack occurrence peak used as an index for the reliability evaluation is determined, a load that gives a peak top of the crack occurrence peak may be used for the reliability evaluation, for example.

In a case where a plurality of crack occurrence peaks satisfying the above threshold value appears, it is preferable to use the crack occurrence peak appearing at the lowest load for the reliability evaluation among the peaks.

The crack occurrence peak suitable as an index for reliability may vary also depending on the position where the indenter is pushed into. Therefore, it is preferable that the position where the indenter is pushed into be determined, a temperature cycle test and the fracture test by the nanoindenter be performed on a plurality of types of test objects in advance under the condition under which the indenter is pushed into the position, and a crack occurrence peak having a high correlation with the temperature cycle test be grasped among the crack occurrence peaks appearing.

Examples

Hereinafter, the present embodiment will be specifically described by providing examples. However, the present embodiment is not limited to the following examples.

Preparation of printed wiring board substrate

Production Examples 1 to 6

Printed wiring board substrates 1 to 6 (substrates each having a thickness of 1.7 to 2.0 mm, with the outer shape being cut out to have a rectangular shape of 60 mm × 60 mm in a plan view) having the following multilayer structure were produced using a build-up material (manufactured by Ajinomoto Co., Inc., product name: "GX92," thickness: 30 $\mu$m) (hereinafter also referred to as "Bu") and copper foil (thickness: 18 $\mu$m or 12 $\mu$m), with a laminated plate obtained by lamination-molding twelve sheets of a predetermined insulating layer forming material (prepreg containing a thermosetting resin composition and a glass cloth) as a core layer. The respective core layers of obtained printed wiring board substrates 1 to 6 are different from one another.

Substrate configurations

Copper foil (18 $\mu$m)/Bu (30 $\mu$m)/Bu (30 $\mu$m)/copper foil (18 $\mu$m)/Bu (30 $\mu$m)/Bu (30 $\mu$m)/copper foil (18 $\mu$m)/Bu (30 $\mu$m)/Bu (30 $\mu$m)/copper foil (18 $\mu$m)/Bu (30 $\mu$m)/Bu (30 $\mu$m)/copper foil (12 $\mu$m)/core layer (1150 to 1350 $\mu$m)/copper foil (12 $\mu$m)/Bu (30 $\mu$m)/Bu (30 $\mu$m)/copper foil (18 $\mu$m)/Bu (30 $\mu$m)/Bu (30 $\mu$m)/copper foil (18 $\mu$m)/Bu (30 $\mu$m)/Bu (30 $\mu$m)/copper foil (18 $\mu$m)/Bu (30 $\mu$m)/Bu (30 $\mu$m)/copper foil (18 $\mu$m)

The thicknesses in the parentheses mean the thicknesses of the respective layers before lamination.

Thereafter, a silicon semiconductor chip (rectangular shape having an outer shape of 20 mm × 20 mm in a plan view and a thickness of 0.775 mm) was disposed at the center of one surface of each of the printed wiring board substrates obtained above so that the circuit surface faced downward, and the gap between the semiconductor chip and the substrate was sealed using a liquid sealing material (Resonac Holdings Corporation, product name "CEL-C-3730 series"), thereby obtaining semiconductor chip mounting substrates 1 to 6 for a liquid bath temperature cycle test.

Liquid bath temperature cycle test

In the liquid bath temperature cycle test, five substrates were prepared for each of the semiconductor chip mounting substrates, and the five substrates were subjected to a temperature cycle test in which heating and cooling were repeated under the following conditions: at -65°C for 5 minutes, at 150°C for 5 minutes, and with a transfer time between a low-temperature bath and a high-temperature bath of 18 seconds. The substrates were taken out at the point when 1,000 cycles had been completed, and a predetermined position of each of the substrates was observed by an optical microscope to confirm the presence or absence of occurrence of cracks.

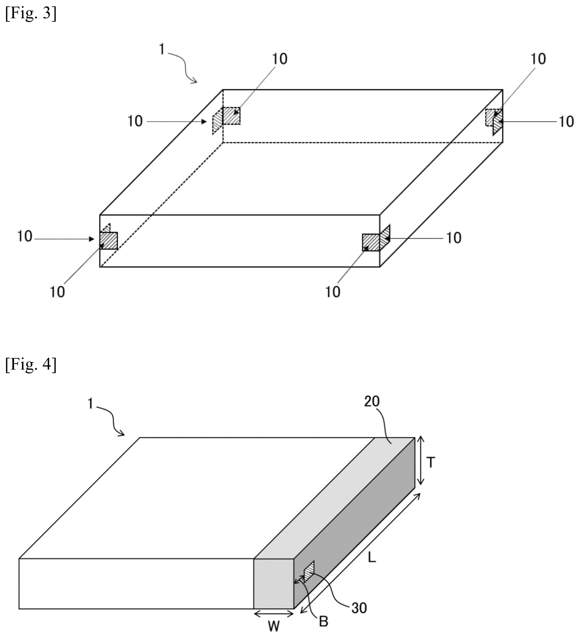

Fig. 3 shows a transparent perspective view for explaining positions observed by an optical microscope. In a substrate 1 shown in Fig. 3, the four corners of the substrate indicated by regions 10 (hatched portions) were each observed from two directions as eight observation positions. The observation range is a region of 1,500 $\mu$m × 1,500 $\mu$m per site.

Each of the five substrates was observed at the above-described eight sites, and the ratio [number of sites where cracks were observed × 100/40 sites] (unit: %) of the sites where cracks were observed among the total of 40 sites was defined as the crack occurrence rate in the liquid bath temperature cycle test.

Fracture test by nanoindenter

Examples 1-6

1. Preparation of test piece

Fig. 4 is a transparent perspective view for explaining the position where the test piece was sampled and the position where the fracture test was performed on the test piece.

Printed wiring board substrates 1 to 6 obtained in Production Examples 1 to 6 were cut, and test pieces each having a size of 1.5 mm (cutting width W) × 60 mm (substrate length L) × 1.7 to 2.0 mm (substrate thickness T) indicated by a portion 20 in Fig. 4 were sampled.

In the sampled test piece, the test region where the fracture test is performed is a region indicated by a region 30 in Fig. 4. The region 30 is a cross sectional surface of an end portion of the printed wiring board substrate 1, and is substantially a central portion of the printed wiring board substrate 1 in a cross sectional view. The distance (distance B in Fig. 4) to the region 30 from the corner of the printed wiring board substrate 1 is about 10 mm.

The cross sectional surface of the printed wiring board substrate including the region 30 was subjected to mechanical polishing using polishing paper of No. 4000 in advance. The obtained test piece was heat-treated at 260°C for 6 hours in an air atmosphere, then cooled to room temperature, and subjected to measurement by a nanoindenter.

2. Fracture test by nanoindenter

The test piece obtained as described above was mounted on the nanoindenter so that the indenter of the nanoindenter was pushed into the test region of the test piece. Thereafter, the position of the indenter was adjusted by an optical microscope and a micrometer attached to the nanoindenter so that the shortest distance between the glass cloth and the center position on the resin cured product where the indenter was pushed into was 5 $\mu$m on the surface into which the indenter was pushed, and the fracture test was performed under the following conditions.

Conditions

Device: product name "DUH-211S" manufactured by SHIMADZU CORPORATION
Type of indenter: Berkovich indenter (with a ridge interval of 115°)
Indentation speed: 39 mN/s
Maximum load: 490 mN
Measurement temperature: 25°C
Measurement atmosphere: in air

3. Analysis of measurement results

Among the crack occurrence peaks appearing on the load-contact stiffness curve obtained by the fracture test described above, a crack occurrence peak having a contact stiffness decrease rate of 37% or more as calculated by the above equation (1) was identified.

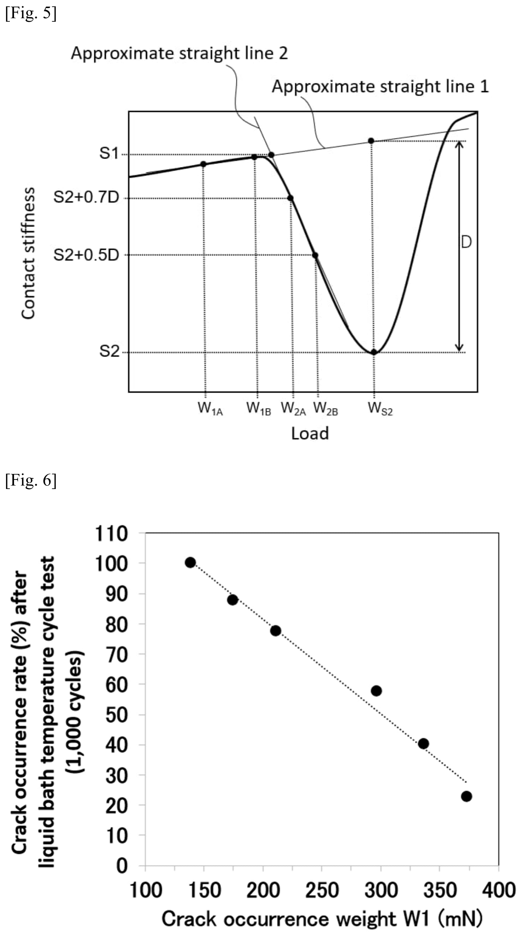

S1 in the above equation (1) was determined by the following procedure. Fig. 5 is a schematic diagram for explaining the method of determining S1.

The contact stiffness that is the peak top of the peak to be analyzed is defined as S2, the load that gives S2 is defined as $W_{S2}$, $W_{S2}$ - 25 mN is defined as $W_{1A}$, and $W_{S2}$ - 15 mN is defined as $W_{1B}$. Next, in the range of $W_{1A}$ to $W_{1B}$, an approximate straight line of the load-contact stiffness curve is obtained by the least squares method, and the straight line is defined as "approximate straight line 1".

Next, the absolute value D of the difference between the contact stiffness S2 and the contact stiffness of the approximate straight line 1 at $W_{S2}$ is calculated, the load that gives the contact stiffness of S2 + 0.7D is defined as $W_{2A}$, the load that gives the contact stiffness of S2 + 0.5D is defined as $W_{2B}$, the approximate straight line of the load-contact stiffness curve in the range of $W_{2A}$ to $W_{2B}$ is obtained by the least squares method, and the straight line is defined as "approximate straight line 2".

The contact stiffness at the intersection of the approximate straight line 1 and the approximate straight line 2 obtained as described above was determined as S1.

Among the crack occurrence peaks having a contact stiffness decrease rate of 37% or more as identified by the above-described method, a crack occurrence peak appearing at the lowest load was identified, and the load giving the peak top of the crack occurrence peak was determined as crack occurrence load W1.

Fig. 6 shows a graph in which crack occurrence load W1 obtained as described above is plotted on the horizontal axis and the crack occurrence rate after the liquid bath temperature cycle test (1,000 cycles) is plotted on the vertical axis, and a linear approximate straight line of the plots obtained by the least squares method.

As is clear from Fig. 6, there is a high correlation between crack occurrence load W1 and the crack occurrence rate after the liquid bath temperature cycle test (1,000 cycles), and it is found that the reliability test method of the present embodiment is useful as a reliability test method for printed wiring board substrates.

Reference Signs List

1 Printed wiring board substrate
10 Observation region
20 Sampled portion of test piece
30 Test region
W Width
L Length
T Thickness
B Distance
S1, S2 Contact stiffness
$W_{1A}$, $W_{1B}$, $W_{2A}$, $W_{2B}$, $W_{S2}$ Weight
A Region

**Claims**

1. A reliability test method for a printed wiring board substrate, the method comprising
measuring a load at which a crack occurs in the printed wiring board substrate, using a nanoindenter.

2. The reliability test method for a printed wiring board substrate according to claim 1, wherein the printed wiring board substrate includes a resin cured product and a glass cloth.

3. The reliability test method for a printed wiring board substrate according to claim 1 or 2, wherein the printed wiring board substrate has two or more layers each including a resin cured product and a glass cloth.

4. The reliability test method for a printed wiring board substrate according to claim 2, wherein an indenter of the nanoindenter is pushed into the resin cured product.

5. The reliability test method for a printed wiring board substrate according to claim 1 or 2, wherein an indenter of the nanoindenter is pushed into a cross sectional surface of the printed wiring board substrate.

6. The reliability test method for a printed wiring board substrate according to claim 1 or 2, wherein a Berkovich indenter is used as an indenter of the nanoindenter.

[Fig. 1]

[Fig. 2]

[Fig. 3]

[Fig. 4]

[Fig. 5]

[Fig. 6]

## INTERNATIONAL SEARCH REPORT

| | International application No. |
|---|---|
| | **PCT/JP2024/014015** |

**A. CLASSIFICATION OF SUBJECT MATTER**

*G01N 3/00*(2006.01)i; *G01N 3/08*(2006.01)i; *G01N 19/00*(2006.01)i
FI:   G01N3/00 T; G01N3/08; G01N19/00 G

According to International Patent Classification (IPC) or to both national classification and IPC

**B. FIELDS SEARCHED**

Minimum documentation searched (classification system followed by classification symbols)

G01N3/00; G01N3/08; G01N19/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | JP 2002-151848 A (MITSUBISHI PLASTICS, INC.) 24 May 2002 (2002-05-24) paragraphs [0001]-[0012], [0038], fig. 1 | 1-4, 6 |
| A | paragraphs [0001]-[0012], [0038], fig. 1 | 5 |
| Y | JP 7-325029 A (NEC CORPORATION) 12 December 1995 (1995-12-12) paragraphs [0001]-[0009], [0018]-[0026], fig. 1-4 | 1-4, 6 |
| A | paragraphs [0001]-[0009], [0018]-[0026], fig. 1-4 | 5 |
| Y | JP 7-260658 A (SHIMADZU CORPORATION) 13 October 1995 (1995-10-13) paragraphs [0007]-[0011], fig. 1-3 | 1-4, 6 |
| A | paragraphs [0007]-[0011], fig. 1-3 | 5 |
| Y | US 6339958 B1 (ADVANCED MICRO DEVICES, INC.) 22 January 2002 (2002-01-22) column 1, lines 5-35, column 3, line 19 to column 5, line 11, fig. 1-9 | 1-4, 6 |
| A | column 1, lines 5-35, column 3, line 19 to column 5, line 11, fig. 1-9 | 5 |

✓ Further documents are listed in the continuation of Box C.    ✓ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | "&" document member of the same patent family |
| "P" document published prior to the international filing date but later than the priority date claimed | |

| Date of the actual completion of the international search | Date of mailing of the international search report |
|---|---|
| **25 April 2024** | **14 May 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

## INTERNATIONAL SEARCH REPORT

International application No.

**PCT/JP2024/014015**

| C. | DOCUMENTS CONSIDERED TO BE RELEVANT |
|---|---|

| Category* | Citation of document, with indication, where appropriate, of the relevant passages | Relevant to claim No. |
|---|---|---|
| Y | JP 2012-146990 A (SUMITOMO BAKELITE CO., LTD.) 02 August 2012 (2012-08-02) paragraphs [0030]-[0037], fig. 9 | 3-4 |
| A | JP 2004-170160 A (NATIONAL INSTITUTE OF ADVANCED INDUSTRIAL SCIENCE AND TECHNOLOGY) 17 June 2004 (2004-06-17) entire text, all drawings | 1-6 |
| A | JP 2001-108586 A (SHIRATORI, Masaki) 20 April 2001 (2001-04-20) entire text, all drawings | 1-6 |

Form PCT/ISA/210 (second sheet) (July 2022)

## INTERNATIONAL SEARCH REPORT
### Information on patent family members

International application No.

**PCT/JP2024/014015**

| Patent document cited in search report | | | Publication date (day/month/year) | Patent family member(s) | Publication date (day/month/year) |
|---|---|---|---|---|---|
| JP | 2002-151848 | A | 24 May 2002 | (Family: none) | |
| JP | 7-325029 | A | 12 December 1995 | (Family: none) | |
| JP | 7-260658 | A | 13 October 1995 | (Family: none) | |
| US | 6339958 | B1 | 22 January 2002 | (Family: none) | |
| JP | 2012-146990 | A | 02 August 2012 | (Family: none) | |
| JP | 2004-170160 | A | 17 June 2004 | (Family: none) | |
| JP | 2001-108586 | A | 20 April 2001 | (Family: none) | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2006093618 A